(19) Europäisches Patentamt
European Patent Office
Office européen des brevets

(11) **EP 3 878 353 B1**

(12) **EUROPEAN PATENT SPECIFICATION**

(45) Date of publication and mention
of the grant of the patent:
**07.06.2023 Bulletin 2023/23**

(21) Application number: **21160670.2**

(22) Date of filing: **04.03.2021**

(51) International Patent Classification (IPC):
*A61B 5/00* *(2006.01)* *G16H 30/40* *(2018.01)*
*G16H 50/20* *(2018.01)* *G16H 50/30* *(2018.01)*
*A61B 6/00* *(2006.01)*

(52) Cooperative Patent Classification (CPC):
**A61B 5/7267; G16H 30/40; G16H 50/20;
G16H 50/30;** A61B 6/50; A61B 6/5217

(54) **CAD DEVICE FOR ASSISTING AN ESTIMATION OF LUNG DISEASE FROM MEDICAL IMAGES**

CAD-VORRICHTUNG ZUR UNTERSTÜTZUNG DER EINSCHÄTZUNG EINER
LUNGENERKRANKUNG BASIEREND AUF MEDIZINISCHEN BILDERN

DISPOSITIF CAD D'AIDE À L'ÉVALUATION D'UNE MALADIE PULMONAIRE À PARTIR D'IMAGES
MÉDICALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2020 US 202016816910**

(43) Date of publication of application:
**15.09.2021 Bulletin 2021/37**

(73) Proprietor: **Optellum Limited
Oxford, Oxfordshire OX1 1BY (GB)**

(72) Inventors:
• **DECLERCK, Jerome**
 **Oxford, Oxfordshire OX1 1BY (GB)**
• **DOWSON, Nicholas**
 **Oxford, Oxfordshire OX1 1BY (GB)**
• **KADIR, Timor**
 **Oxford, OX1 1BY (GB)**
• **POTESILI, Vaclav**
 **Oxford, OX1 1BY (GB)**

(74) Representative: **Optimus Patents Limited
Peak Hill House
Steventon
Basingstoke, Hampshire RG25 3AZ (GB)**

(56) References cited:
**US-A1- 2010 063 410 US-A1- 2016 130 656**

• **GURNEY J W ET AL: "SOLITARY PULMONARY
NODULES: DETERMINING THE LIKELIHOOD OF
MALIGNANCY WITH NEURAL NETWORK
ANALYSIS", RADIOLOGY, RADIOLOGICAL
SOCIETY OF NORTH AMERICA, INC, US, vol. 196,
no. 3, 1 September 1995 (1995-09-01), pages
823-829, XP009001279, ISSN: 0033-8419**

## Description

Field of the invention

The field of this invention relates to computer-aided diagnosis (CADx) device for assisting an interpretation of medical images in order to support clinicians in healthcare. In particular, it relates to a device for estimating a likelihood of lung disease by inferring smoking history from a medical image. Background of the invention

Lung cancer remains the most common cause of cancer-related death in the UK and USA, even though lung cancer is usually curable if caught at an early stage. As a result of recent lung cancer studies, including the National Lung cancer Screening Trial (NLST) and the Dutch-Belgian Randomized Lung Cancer Screening Trial (with the Dutch acronym NELSON), the large scale screening of patients using Computed Tomography (CT) is now being considered for roll-out by national health organisations. For example, in some areas of the UK, the National Health Service (NHS) is currently enrolling over 55s who are ex-/current smokers for the 'Lung Health Check' program. In the program, those people with abnormally low lung function, say as assessed by spirometry, receive a CT scan. A CT scan is performed by a machine that analyses how much X-Rays are absorbed by the body when emitted from different angles to generate the CT, which is a three-dimensional (3D) reconstruction of the different tissues of the patient. The CT can also be referred to as a medical image, although this term is broader, in that it can also refer to images generated by other scanners, such as Magnetic Resonance Imaging (MRI), Positron Emission Tomography (PET), which are also sometimes used to aid in diagnosing lung cancer. In addition to screening programs, CT scans are often taken of the chest to check for broken bones or investigate the causes of the symptoms of disease, such as a persistent cough, shortness of breath, chest pain or fever. In addition to any other diseases such as Bronchiectasis or Chronic Obstructive Pulmonary Disease (COPD), the CT is also examined to check for lung nodules. Patients in whom suspicious lung nodules are identified then undergo a biopsy or follow-up imaging, in order to check whether the lung nodules are cancerous.

The increasing number of chest CTs that need to be examined for suspicious lung nodules is a challenge because it relies on each CT being manually assessed by an expert radiologist. To assist in the efficient and accurate examination of CTs, Computer Aided Diagnosis (CADx) devices can be used to aid in the diagnosis of detected abnormalities. CADx devices typically operate by relying on the user to identify abnormalities within the image for analysis, and then performing a series of mathematical operations on an array of intensities at the location of the abnormalities. For example, if a radiologist has identified a lung nodule in a CT scan, the CADx device can provide a score that is indicative of a risk of malignancy. The score is computed by a component of the CADx device, referred to hereinafter as the CADx system that may be a part of a machine that generates the score. That score indicates a risk or likelihood of disease, or its absence. An example of a commercial CADx device is the Transpara™ product from Screenpoint™ for breast cancer characterisation from X-Ray mammograms.

There are many examples of such CADx devices proposed within the academic literature. For example, CADx devices that use neural networks is described in "Solitary pulmonary nodules: determining the likelihood of malignancy with neural network analysis" (by Gurney et al, 10.1148/radiology. 196.3.7644650) to differentiate benign and malignant solitary pulmonary nodules. In this paper, it is described that the neural network analysis is based on five identified radiographic characteristics of the nodules (edge, size, location, calcification, growth) and two recorded clinical characteristics of the patient (age, smoking history in pack-years).

However, very few systems are available commercially, and hence used in clinical practice. This discrepancy is indicative of the difficulties in deploying practical systems with the known approaches.

CADx devices are developed using data collected during clinical studies. Since cancer is rare, it is necessary that data is typically collected from high risk groups of people, for instance in NLST only data from over-55 years old ex-smokers was used. The collection of data from low-risk groups is usually not possible for ethical reasons, as CT scanning comes with a small increase in a risk of cancer, and would require unfeasibly large study cohorts. Hence, in order to interpret the output of the CADx device effectively and make the correct decision about their patient, doctors need additional information about the patient. For lung diseases, smoking history is particularly salient, because smoking damages the lung tissue, thereby increasing the risk of disease. Knowing the smoking history is especially important to non-radiologist clinicians involved in decision making for lung nodules, such as pulmonologists and also junior radiologists, because they lack the expertise of a radiologist who has analysed thousands of chest CTs over many years. Smoking history is typically reported as a number of pack-years. Pack-years is defined as the number of years a person smoked multiplied by the numbers of packs per day they smoked. For instance someone who smoked one pack of cigarettes per day for 10 years will have a 10 pack-year history, as will someone who smoked two packs of cigarettes per day for 5 years.

However, smoking history is not typically reported unless the CT was specifically taken in the context of a screening program. Smoking history is often difficult or impossible to obtain at a later stage. Even if smoking history is reported, it is often inaccurate for reasons such as patient embarrassment or lapses in memory. As a result, the inventors have identified and appreciated that when non-radiologist clinicians or junior radiologists review images using a CADx device, too much credence is granted to the score output by the CADx device. In particular, the CADx score for nodules of long-term smokers, whose smoking history is not known or under-reported, will under-estimate the risk of cancer, with the potential result of the patient being incorrectly discharged and the cancer remaining undiscovered until years later when it has become incurable. In contrast, patients who have never smoked may have too high a risk associated with their nodules, and undergo unnecessary follow-up CTs, or even an unnecessary biopsy with the corresponding medical risk and discomfort associated with a biopsy procedure.

Thus, the inventors have recognised and appreciated that there exists a desire for an improved Computer Aided Diagnosis (CADx) device to assist the reading and reporting of medical images by radiologists and the interpretation of the radiologist's report by the physician responsible for patient care, the so-called 'referring physician'.

Summary of the invention

The invention is defined in the independent claim 1. Preferred embodiments are set forth in the dependent claims.

Accordingly, the invention seeks to mitigate, alleviate or eliminate one or more of the abovementioned disadvantages singly or in any combination.

According to an aspect of the present invention, a Computer Aided Diagnosis, CAD, lung disease risk measure device is described that comprises: an input circuit configured to receive at least one input medical image of a patient in which the patient's lungs are visible. A smoking history system is operably coupled to the input circuit and configured to receive and analyse the at least one input medical image of the patient and determine an equivalent smoking history of the patient based on the at least one input medical image and output a smoking history parameter based on the determined equivalent smoking history of the patient. A CADx system is configured to receive and analyse the smoking history parameter and the at least one input medical image, and calculate and output a lung disease risk measure based on a combination of the analysis of the at least one input medical image and the smoking history parameter. An output circuit is operably coupled to the smoking history system and the CADx system is arranged to output the lung disease risk measure.

In this manner, the smoking history system may provide supplementary information to a CADx device in order to create a modified indication of a patient's risk of lung cancer.

In some optional examples, the smoking history system may determine an equivalent smoking history of the patient by comparing the at least one input medical image with a range of pre-stored, classified input medical images, and may determine the smoking history parameter based on a quantification of cumulative damage of the patient's lungs as analysed from the at least one input medical image.

In some optional examples, the CADx system may further comprise an input configured to receive at least one clinical factor related to the patient and calculate and output a lung disease risk measure based on a combination of the analysis of the at least one input medical image and the received smoking history parameter and the at least one clinical factor of the patient.

Such a CADx device, CADx system and/or method to analyse a patient's medical images and accommodate one or more other clinical factors in the analysis and generation of results may provide critical context for clinicians (and automatic systems) assessing patients at risk of lung disease, as well as patients who have suspicious lung masses. This then allows the clinician to better select when further surveillance or treatment of the patient is required. Furthermore, employing examples of the invention will ensure fewer diseases have a delayed diagnosis, and fewer patients undergo unnecessary treatments or invasive biopsies.

In some optional examples, the CADx lung disease risk measure device may further comprise a CADx system configured to receive and analyse the at least one input medical image and calculate and output a lung disease risk measure; a risk score adjustment system coupled to an output of the CADx system and an output of the smoking history system and configured to adjust the received lung disease risk measure based on the received smoking history parameter of the patient; and an output circuit operably coupled to the risk score adjustment system and arranged to output a modified lung disease risk measure. In some examples, the risk score adjustment system may further comprise an input configured to receive at least one clinical factor related to the patient and configured to adjust the received lung disease risk measure based on the received smoking history parameter and the at least one clinical factor of the patient.

In some optional examples, the at least one clinical factor may comprise data on at least one of: the patient's physical age, the patient's body-mass index, a diagnosed condition, a clinical measure of lung function, whether the patient is currently suffering from an infection. In some examples, either the CADx system or the risk score adjustment system may be configured to automatically compute an effective lung damage of the patient based on the at least one medical image and map the effective lung damage of the patient to a measure of a damaged lung that is equivalent to a second patient having a smoking history. In some examples, the lung disease risk measure device may be configured to generate the measure of the damaged lung in terms of a period of time and a quantity of smoking during that time of an equivalent smoking patient.

In some optional examples, the lung disease risk measure may be a single number that provides a modified risk measure of the patient's risk of lung disease beyond a natural effect of patient aging.

In some optional examples, at least one of: the CADx system and the smoking history system and the risk score adjustment system may be implemented as a neural network. In some optional examples, at least two of: the CADx system the smoking history system and the risk score adjustment system may be as a neural network and they may be trained jointly, for example where the neural network may be a convolutional neural network trained using a set of Computed Tomography, CT, images of patients with associated labels that define their smoking history in pack-years.

In some optional examples, if an existing CADx system is to be adapted without change to its internal workings, an equivalent smoking history may be used to modify an existing lung cancer risk score by a risk score adjustment system to provide a more accurate lung cancer risk score. In some optional examples, clinical factors of the patient such as physical age or body mass index can be provided as additional and/or optional inputs to the risk score adjustment system in order to provide a more accurate lung cancer risk score.

In some optional examples, the at least one input medical image may comprise an associated at least one input ground-truth label and wherein at least one of: the CADx system and the smoking history system and the risk score adjustment system may be configured to calculate a difference between the determined equivalent smoking history of the patient and a corresponding at least one input ground-truth label using a loss function. In some examples, the at least one input ground-truth label may comprise at least the actual smoking history of the patient and a difference between the smoking history parameter and the actual smoking history may be computed using a loss function during training.

These and other aspects of the invention will be apparent from and elucidated with reference to the embodiments described hereinafter.

Brief description of the drawings

Further details, aspects and embodiments of the invention will be described, by way of example only, with reference to the drawings. In the drawings, like reference numbers are used to identify like or functionally similar elements. Elements in the figures are illustrated for simplicity and clarity and have not necessarily been drawn to scale.

FIG. 1 illustrates an example of a current state-of-the art CADx device being used to estimate lung cancer for two examples of medical images in order to demonstrate the shortcomings of the current state-of-the-art as identified by the inventors of the present invention.

FIG. 2 illustrates how the knowledge of smoking history can lead to an improved estimate of the risk of lung disease, such as lung cancer.

FIG. 3 illustrates an example of a CADx device to automatically calculate smoking history in order to better estimate the risk of lung disease, such as lung cancer according to examples of the present invention.

FIG. 4 illustrates an example of a smoking history system to automatically calculate an equivalent smoking history from a medical image using a model based on intensity histograms.

FIG. 5 illustrates an alternative example of a smoking history system to automatically calculate an equivalent smoking history from a medical image using a more complex model, such as a neural network, that requires the model parameters to be generated using a training algorithm.

FIG. 6 illustrates an example of a neural network that may be employed as a model architecture to estimate smoking history according to some examples of the present invention.

FIG. 7 illustrates how the smoking history system can be used to improve an existing CADx device, for example an existing CADx system within the CADx device whose internal functionality cannot be changed.

FIG. 8 illustrates an example of how the smoking history, apparent from a medical image, relates the physical age of a patient, even if they have never smoked.

FIG. 9 illustrates an example of how the equivalent smoking history for one patient can be referenced against a spectrum of equivalent smoking histories for patients of a similar age or those patients who are older and/or younger and to identify when an individual patient is at increased risk of cancer.

FIG. 10 illustrates an alternative example of a CADx device in order to automatically calculate smoking history to better estimate the risk of lung disease, such as lung cancer, by being trained to optionally and additionally output clinical factors according to some examples of the present invention.

Detailed description

The inventors have recognised and appreciated that there exists a desire for an improved Computer Aided Diagnosis (CADx) device to assist the reading and reporting of medical images by radiologists and the interpretation of the radiologist's report by the physician responsible for patient care, the so-called 'referring physician', by incorporating a system to determine a lung condition that is equivalent to another patient with a smoking history. Examples of this invention propose an approach to resolve the issues of lack of relevant and accurate information on clinical factors related to estimating the risk of lung disease, such as lung cancer for a patient by a CADx device being configured to estimate the risk of cancer directly from the medical image, together with an assessment of an equivalent smoking history of the patient.

In examples of the invention, a CADx device is generally used to refer to the device that analyses the image data that is used to assist the reading and reporting of medical image by radiologists and the interpretation of the radiologist's report by the physician responsible for patient care. In examples of the invention, a CADx system is generally used to refer to the component that generates a lung condition (e.g. lung cancer) score. In examples of the invention, a smoking history system is generally used to refer to the component that generates the smoking history parameter.

In some examples, a CADx system may be used to output data or a result/value that provides a risk of a patient having a lung condition from medical data images and the smoking history of the patient. Hence, references to a CAD training system or computer-aided risk assessment system hereafter encompasses a CADx system or a risk model that is configured to identify a risk of a patient having such a medical condition.

A longer history of smoking implies an increased risk of (lung) conditions or diseases, such as lung (and other) cancers. Hence, an automatic assessment of an equivalent smoking history can provide an objective factor that can be used to improve accuracy when predicting the likelihood of nodule malignancy, for instance. Such a measure can also provide further context to physicians reviewing the CT images.

Clinicians face the further challenge of identifying people who have a high risk of lung disease and ought to be screened, despite never having smoked, for instance those people who have had extended sojourns within cities with high pollution levels, had industrial exposure to chemicals, such as asbestos, or resided in regions where lung infections from endemic fungal flora are common. Thus, the inventors have recognised a further desire for a system to help clinicians to identify non-smoking patients at risk of lung disease for screening by using a system to determine a lung condition that is equivalent to another patient with a smoking history.

In the case of smokers whose smoking status increases their risk of lung disease, but whose smoking history is not known to the clinician, there is also a need to identify them for smoking cessation advice. Thus, the inventors have recognised a further desire for a system to help clinicians identify smokers in need of smoking cessation advice by using a system to determine a lung condition that is equivalent to another patient with a smoking history.

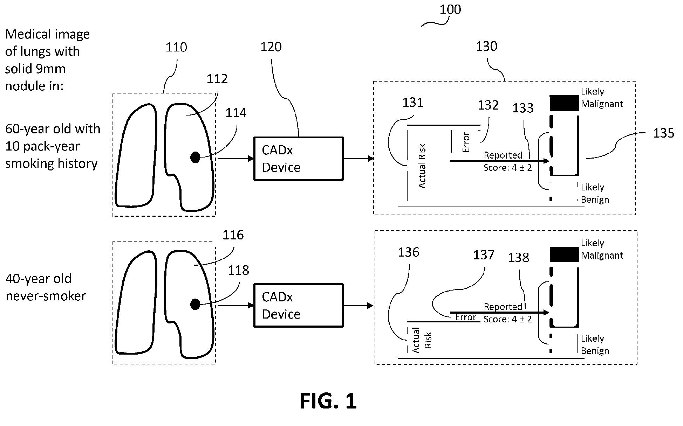

Referring first to FIG. 1, a schematic of an existing state-of-the-art CADx device 120 for assessing the risk of lung cancer is shown. Such a CADx device 120 may take at least a medical image 110 as an input and provide a risk score associated with the cancer 130.

However, in order to better appreciate the concepts herein described, let us consider now the example of the chest CTs of two patients. The first CT 112 is of a 60-year old with a 10 pack-year smoking history with a solid 9mm nodule 114. The CADx device 120 outputs a cancer risk score 133 of $4\pm2$ on a scale 135 of, say, zero to ten, where the numeral '4' represents the estimated risk and '$\pm2$' represents the uncertainty associated with the cancer risk score 133. The cancer risk score 133 is an estimate of the risk of cancer associated with solid 9mm nodules, such as the one in the CT. However, and notably, the inventors of the present invention have identified that this cancer risk score 133 will differ from the actual underlying (more accurate) risk 131, because accurate information on the patient's smoking history is not available to the CADx device 120. Hence, there is a negative error 132, which results in the score underestimating the risk of cancer, thereby increasing the chances that a cancer is missed and will go untreated.

The second CT 116 is of a 40-year old who has never smoked and who also has a solid 9mm nodule 118 that exactly resembles the smoker's nodule 114. Since the two nodules resemble each other the CADx device 120 also outputs a score of $4\pm2$, for the never-smoker's nodule. However, in the never-smoker's case the actual underlying (more accurate) risk 136 of cancer is lower than that for the smoker 131 and the error 137 is positive because the CADx device does not have an accurate smoking history. Hence, the reported score for the never-smoker 138 is an over-estimate of their actual risk of cancer, thereby increasing the chances that the patient will undergo an unnecessary follow-up CT or a biopsy surgery procedure.

In this manner, FIG. 1 serves to highlight the inventors of the present invention's identification as to why an improved (more accurate) estimate of smoking history is important for CADx devices, in assessing a risk of lung disease, such as lung cancer.

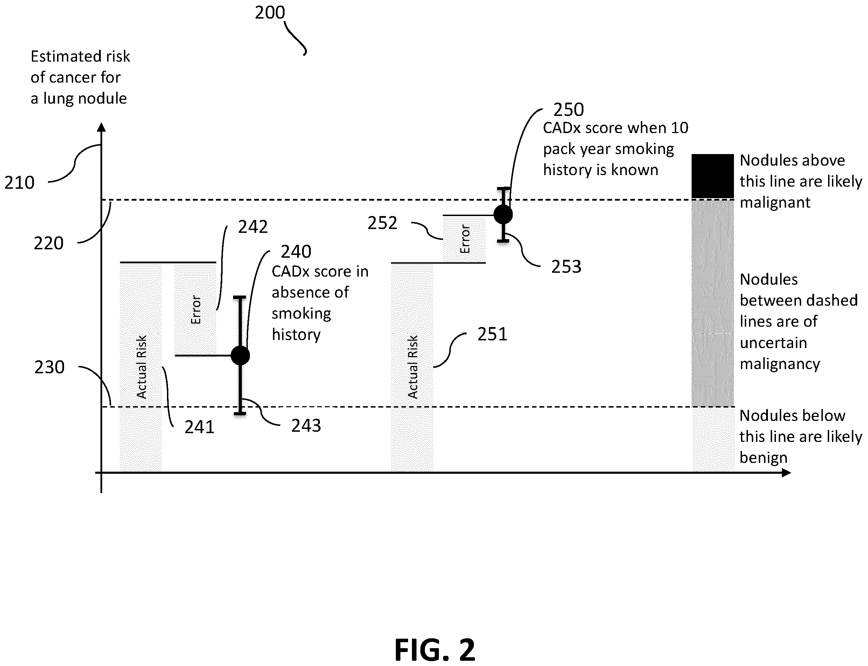

Referring now to FIG. 2, and using the previous example of the 9mm nodule in a patient with a 10 pack-year smoking, the estimated risk of cancer for a nodule output 210, provided by two CADx devices (or the same CADx device at two different instances of time) are shown. For example, CADx scores above an upper threshold 220, are considered to be likely malignant, while scores below a lower threshold 230 are considered to be likely benign. The CADx score 240 from a current state-of-the-art CADx device comprises of the intrinsic underlying risk 241. Notably, the CADx score 240 also includes an error margin 242, where the margin is due to factors, such as smoking history, that the CADx device does not know with any accuracy, if at all. As a result, the uncertainty 243 associated with the score is relatively large.

When being assessed using a CADx device that incorporates an automatic measure of smoking history, as proposed in examples of this invention, the intrinsic underlying risk 251 is the same, but because an accurate smoking history is utilised, the error margin 252 can be advantageously, and sometimes significantly, reduced and the uncertainty 253 in the score 250 can also be reduced.

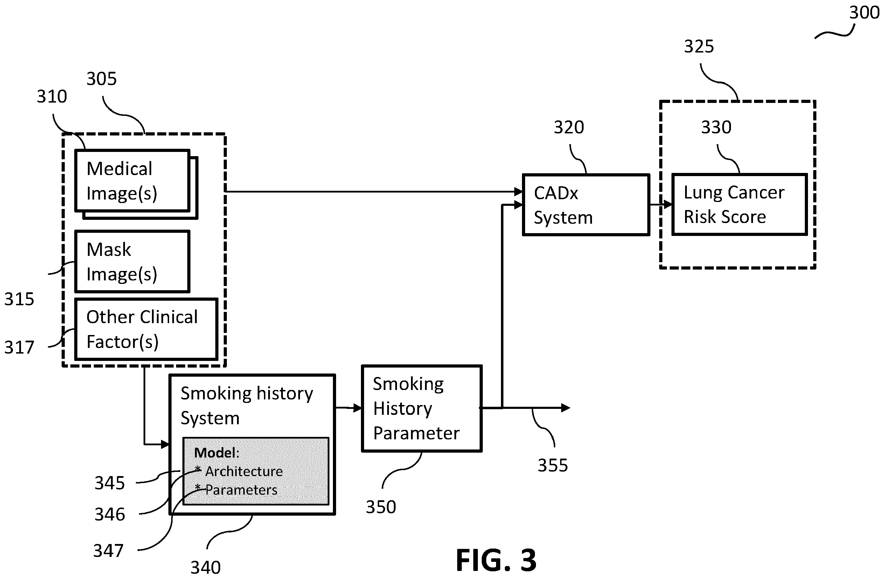

Referring now to FIG. 3, an example of a CADx device 300 is illustrated to better determine a risk of a lung disease, such as lung cancer, according to examples of the present invention. The CADx device 300 comprises a smoking history system 340 operably coupled to an input circuit 305, which takes at least one medical image, such as a thoracic CT image 310 of a patient in which the patient's lungs are visible, as an input, and outputs a smoking history parameter 350. The CADx device 300 further comprises a CADx system 320 operably coupled to the input circuit 305 and receives and processes the at least one medical image and the smoking history parameter 350 as inputs.

The at least one input medical image comprises of a plurality of intensities, which in the case of a CT image represent the opacity of the tissues visible within the image to X-Rays. In this example, the smoking history system 340 includes a representation of a mathematical model 345. The mathematical model 345 itself comprises of a set of instructions for performing a series of mathematical operations that are performed in a particular order on the intensities in the input image, in order to calculate the smoking history parameter 350, which are referred to as the model architecture 346. The model also includes a collection of numbers, referred to as model parameters 347, which are used by the model architecture in its calculations. An output circuit 325 is operably coupled to the smoking history system 340 and the CADx system 320 and is arranged to output a lung disease risk (e.g. cancer) measure/score 330 based on a combination of the analysis of the at least one input medical image 310.

Optionally, in some examples, at least one mask image 315 may be applied, which defines those regions of the thoracic CT image(s) where the lungs lie. Thus, in one illustrated example, such a mask image 315 may also be supplied as an input to the smoking history system 340. The at least one mask image 315 serves to help the smoking history system 340 exclude non-lung regions from consideration.

Optionally, in some examples, at least one clinical factor other than smoking history 317 that is associated with the patient, for example whether the patient currently has a lung infection, body-mass index, physical age, diagnosed conditions for instance emphysema or asthma, and clinical measures of lung function, may be provided as inputs supplementary to the medical image(s) that also enable a lung cancer risk score to be calculated.

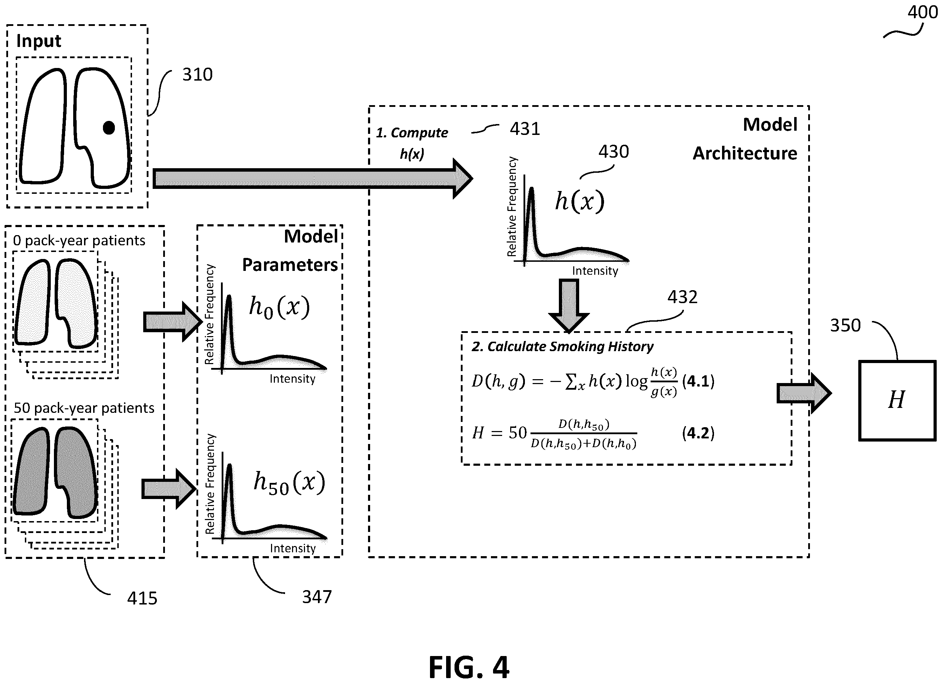

It is envisaged that the smoking history system can be implemented in a number of ways. Referring now to FIG. 4, in some examples of the invention 400, the model parameters of the smoking history system 347 may consist of two histograms that describe the relative frequency of intensity within the lungs within a set of training data 415 comprising of medical images for patients who have never smoked, $h_0$, and patients who have, say, a 50 pack-year smoking history, $h_{50}$. From the input medical image 310, the model architecture of the smoking history system includes instructions 431 to compute the intensity histogram, $h(x)$, 430. The smoking history system may then use further instructions 432 to calculate the Kullbach-Liebler Divergence, $D_0$, (4.1) between $h(x)$ & $h_0(x)$, which is a measure of how similar the two histograms are.

Kullbach-Liebler Divergence:

$$D(h, g) = -\sum_x h(x) \log \frac{h(x)}{g(x)} \qquad (4.1)$$

In some examples, the Kullbach-Liebler Divergence between $h_{50}$ and $h$ is also computed.

In some examples, the smoking history system 415 may then compute the smoking history parameter 350, $H$, using smoking history ratio (4.2).

Smoking history ratio:

$$H = 50 \frac{D(h, h_{50})}{D(h, h_{50}) + D(h, h_0)} \qquad (4.2)$$

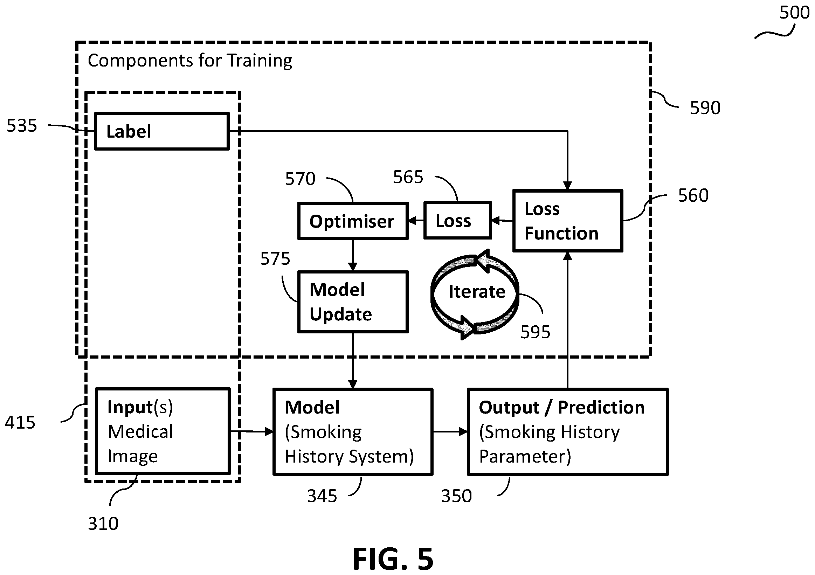

Referring now to FIG. 5, in accordance with some examples of the invention, the model parameters for the smoking history system 345 need to be obtained using a training algorithm in order for the input 310 to generate a predicted output 350. The training process 595 relies on additional training components 590 and an input ground-truth label 535 corresponding to each example input medical image 310. The input medical image(s) 310 and the corresponding input ground-truth label(s) 535 are provided together by the input circuit 415. In some examples of the invention, the training of the model smoking history system 345 may entail repeatedly presenting one or more input medical image(s) 310 to the model smoking history system 345, in order to obtain a prediction for each output 350 of FIG. 5. In some examples of the invention, the difference between the at least one prediction output 350 and the corresponding at least one input ground-truth label 535 associated with each at least one input medical image 310 may be computed using a loss function 560. An example of a loss function is shown in equation (5.1):

Example loss function:

$$L = X(\hat{H}, H) \tag{5.1},$$

where the loss function, $L$, computes a mathematical function called the negative cross-entropy, $X$, between the actual smoking history, $\hat{H}$, and smoking history parameter, $H$. As those skilled in the art will appreciate, other loss functions could be used, for instance the $L_1$-nom function or the sum of square differences.

In some examples of the invention, an optimisation algorithm 570 may be used to calculate a change in the model parameters, referred to as a model update 575 that would reduce the loss 565 output by the loss function 560, for example by performing a mathematical operation called differentiation. Following a model update, the examples can again be presented as an input to the model in order to compute an update. Each cycle of calculating a loss 565, calculating a model update 575 and updating the model is referred to as an iteration. The data used to update the model is referred to as the training set.

As those skilled in the art will recognise, the training data may be selected to comprise of patients of equal numbers of examples across a range of ages and across a range of smoking histories, in order to ensure the equivalent smoking history is accurately estimated, whatever the age of the patient. For instance, the training data may be selected to include, say, 600 patients: 100 patients each at ages 45, 50, 55, 60, 65, and 70 years, and 100 patients each with a smoking history of 0, 5, 10, 15, 20, and 25 pack years. Similarly, to account for the differing effects of cigarette brand, cigarette filter type, cigar-smoking versus cigarette smoking, intensity of smoking and other influences on lung disease risk, the training data can be selected to include data with different examples of each type of influence.

In some examples of the invention, the loss 565 is also computed for a second set of input data and corresponding input ground-truth labels, but where model updates are **not** performed. This is referred to as the validation set. The validation set can be used to decide when to terminate a training process, for example when the loss 565 for the validation set no longer decreases. After the training of the model is complete, the model is able to estimate the smoking history from the input without the need for the components employed to train the model 590, and these components 590 can be discarded after the training process has completed.

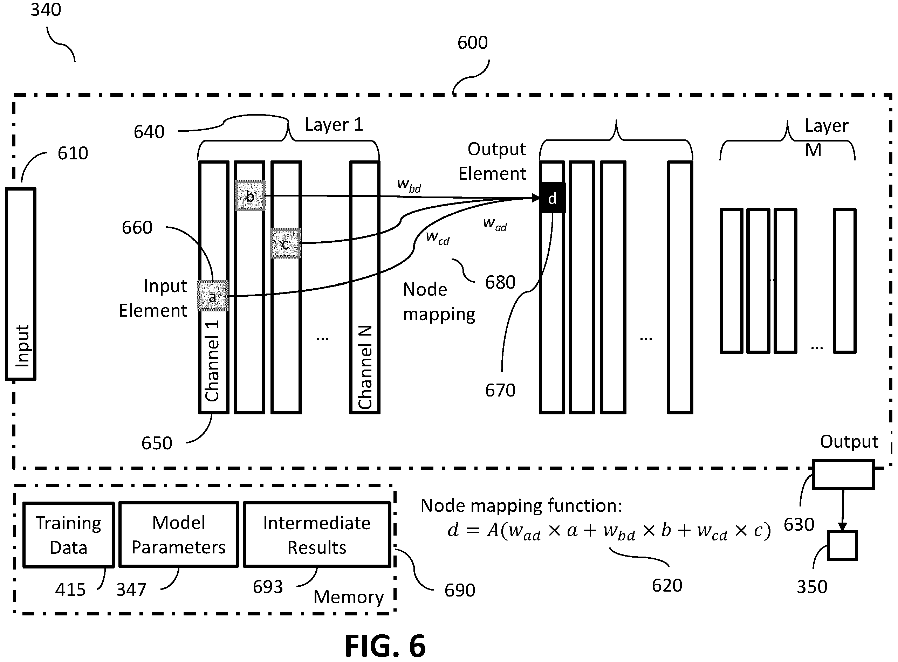

Referring now to FIG. 6, the model architecture with a smoking history system may, for example, be the illustrated neural network, according to examples of the present invention. In some examples, the smoking history system 340 may comprise a convolutional neural network 600, which applies a series of node mappings 680 to an input 610, which ultimately resolves into an output 630 consisting of one or more values, from which at least one of the values is used by the CADx system 320, for example a smoking history parameter 350 of FIG. 3. The input layer may, for example, include the intensities of the input medical image 310, and the mask image 315 and the clinical parameters other than smoking history 317. The example convolutional neural network 600 comprises a consecutive sequence of network layers (e.g. layers 640), each of which consists of a series of channels 650. The channels are further divided into input elements 660. In this example, each input element 660 stores a single value. Some (or all) input elements 660 in an earlier layer are connected to the elements in a later layer by node mappings 680, each with an associated weight. The collection of weights in the node mappings 680, together, form the model parameters. For each node mapping 680, the elements in the earlier layer are referred to as input elements 660 and the elements in the output layer are referred to as the output elements 670. An element may be an input element to more than one node mapping, but an element is only ever the output of one node mapping function 620.

In order to calculate the output 630 of the convolutional neural network 600 the system first considers the input layer as the earlier layer. The layer(s) to which the earlier layer is connected by a node mapping function 620 are considered in turn as the later layer. The value for each element in later layers is calculated using the node mapping function 620 in equation (6.1), where the values in the input elements 660 are multiplied by their associated weight in the node mapping function 620 and summed together.

Node mapping function 620:

$$d = A(w_{ad} \times a + w_{bd} \times b + w_{cd} \times c) \tag{6.1}$$

The result of the summing operation is transformed by an activation function, 'A' and stored in the output element 670. The convolutional neural network 600 now treats the previously considered later layer(s) as the earlier layer, and the layers to which they are connected as the later layers. In this manner the convolutional neural network 600 proceeds from the input layer 640 until the value(s) in the output 630 have been computed.

In examples of the invention, the convolutional neural network 600 may be trained using a set of CT images of patients with associated labels that, say, define their smoking history in pack-years. In some examples of the invention, the training of the convolutional neural network 600 may entail repeatedly presenting at least one CT image to the input 610 of the convolutional neural network 600, in order to obtain the estimated smoking history parameter 350 of FIG. 3, for example by following a process 595 and using training components 590 analogous to those in FIG. 5. In some examples of the invention, the difference between the estimated smoking history parameter 350 and the actual smoking history may be computed using a loss function. In some examples of the invention, an optimisation algorithm may be used to reduce the loss, for example by measuring how much each node mapping 680 weight contributed to the loss, and using this to modify the node mapping functions 620 in such a way as to reduce the loss. Each such modification is referred to as an iteration. After a sufficient number of iterations the convolutional neural network 600 can be used to estimate a patient's smoking history from an input of any one or more CT image(s).

In some examples of the invention, the large number of parameters used in the convolutional neural network may require the device to include a memory 690. The memory 690 may be used to store the training data 415, the model parameters 347, and the intermediate results of the node mappings 693.

If the convolutional neural network 600 is trained by presenting examples whose geometric scale is optionally encoded at the input 610, for example by storing the physical coordinates of each image voxel or by providing the voxel size as a separate input, then the convolutional neural network 600 will retain a notion of scale, in order to account for the physical dimensions of the patient when measuring/estimating smoking history parameter 350. Likewise, in some examples, if the intensity of the image intensities are optionally encoded such that each intensity value of the image has a one-to-one mapping to Hounsfield Units, then the convolutional neural network 600 can estimate the smoking history parameter 350 by examining its effects that manifest as visible changes in the CT intensities.

In some examples, the trained convolutional neural network 600 can then be applied to patients who have smoked for a different number of pack-years, in order to estimate their smoking history.

In some examples, another neural network can comprise the CADx system, which may differ from the neural network in the smoking history system in architecture but still operate using the same principles. Hence, while the above description of a neural network refers to the smoking history system, a skilled artisan will readily appreciate that an analogous approach can be used to construct a CADx system, such as CADx system 320 in FIG. 3. In some examples, it is envisaged that the CADx system 320 and the smoking history system 340, if both implemented as neural networks, can be trained independently or jointly.

Those skilled in the art will readily appreciate that the smoking history system 340 can be implemented as a hardware device, a software package in a general purpose computer or on a firmware device, such as a DSP.

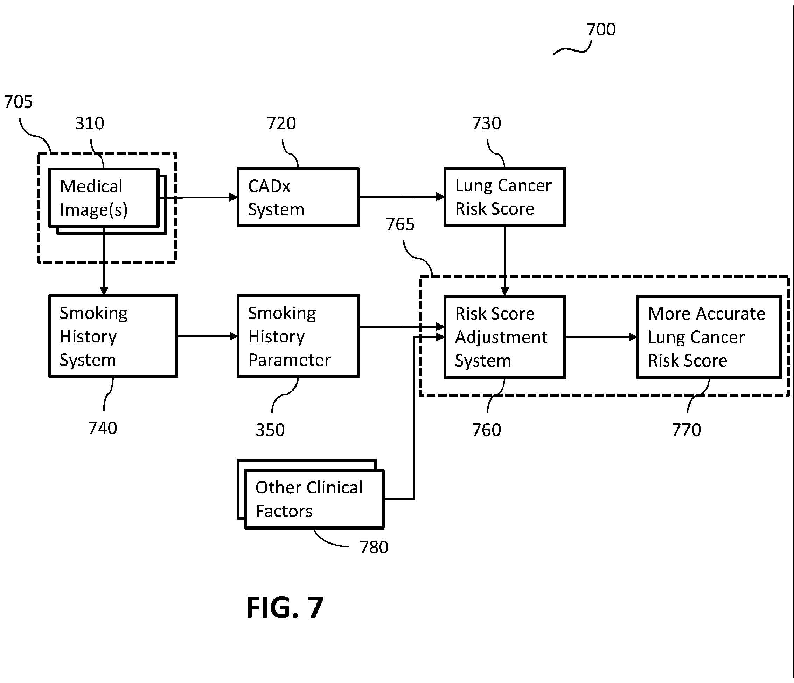

Referring now to Fig. 7, a further example of a CADx device 700 is illustrated, where a smoking history system 740 may be combined with an existing CADx system 720 for estimating a risk of lung cancer whose internal workings may not be changed, with such a system operably coupled to an input circuit 705 receiving at least one medical image as an input 310 and outputting a lung cancer risk score 730. The lung cancer risk score 730 and the smoking history parameter 350 may be coupled to an output circuit 765 comprising a risk score adjustment system 760, which is a processor configured to adjust the uncorrected lung disease risk score 730, such as lung cancer risk score using the smoking history parameter 350, in order to calculate a more accurate lung disease risk score 770. One example of how the lung disease risk score may be adjusted is by performing a series of operations as defined by a mathematical model, such as that in equation (7.1):

Risk score adjustment model 760:

$$\hat{R} = R + w_h \times (H - \mu_h) \qquad (7.1),$$

where $R$ is the uncorrected lung disease risk score 730,
$H$ is the smoking history parameter 350,
$\mu_h$ is the average smoking history of all patients in the training data, and,
$w_h$ a weight the controls effect how much a smoking history parameter increases or decreases $R$.

The weight $w_h$ may be found, for example, using the optimisation process described in FIG. 5. As those skilled in the art will recognise, mathematical models other than (7.1) can be used in the device 700.

Thus, the risk score adjustment system 760 of the output circuit 765 is operably coupled to the smoking history system 340 and the CADx system 720 and is configured to output a more accurate lung disease (e.g. cancer) risk measure/score 770 based on a combination of the analysis of the at least one input medical image 310.

In some examples, the risk score adjustment system may also accept clinical factors other than smoking history 780 as optional and additional inputs, where such clinical factors 780 have a bearing on the risk of lung disease and could further reduce the error in the more accurate lung cancer risk score 770. In some examples, the clinical factors 780 may include physical age, whether the patient currently has a lung infection, body-mass index, diagnosed conditions for instance emphysema or asthma, and clinical measures of lung function.

In some examples, it is envisaged that the CADx device 700 can be applied to estimating a risk of any disease involving the lungs, including lung cancer and Chronic Obstructive Pulmonary Disease, dependent on the one or more input(s) medical image(s) 310 and may include one or more clinical factor(s) that the CADx system uses.

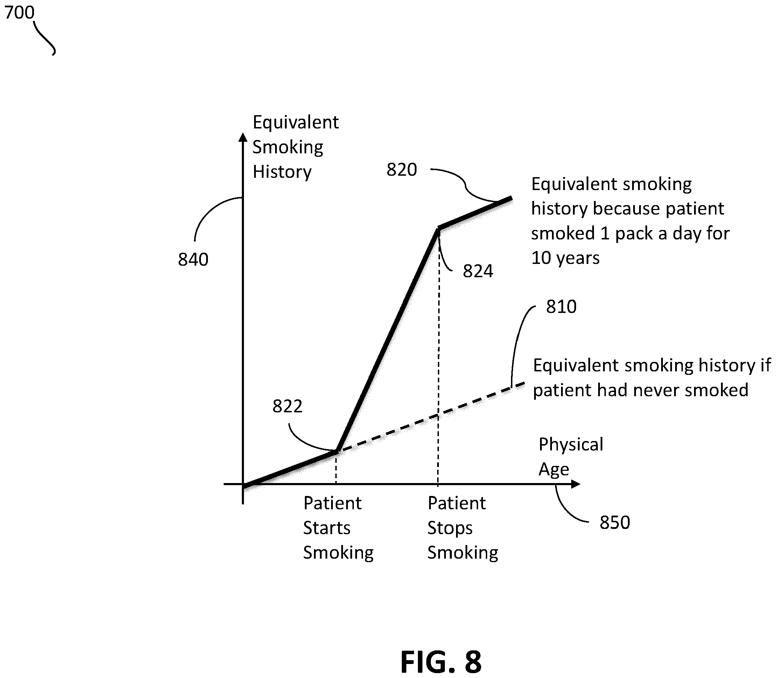

In some examples, it is envisaged that the determined smoking history parameter may be useful for clinicians in its own right. FIG. 8 illustrates an example of how even a patient who has never smoked may have a non-zero equivalent smoking history 810, because the natural effects of aging result in some damage to the lungs that manifests in CTs in the same way as the damage from, say, light smoking, i.e. equivalent smoking history 840 increases as physical age 850 increases.

The visible manifestations of the damage to the lungs are a surrogate measure of the accumulated damage to the cells within the lungs and their DNA that has occurred due to successive biological insults that have occurred over a patient's lifetime. Biological insults may occur due to extended sojourns in cities that suffer from high levels of pollution, repeated exposure to industrial chemicals such as asbestos or inflammatory responses to lung infections for instance. Smoking will also cause damage. Thus the smoking history parameter can be treated as a measure of equivalent smoking history, i.e. the observed damage is equivalent to having smoked for a certain length of time. The damage to the DNA of cells within lung can result in the mutations that lead to cancer. One of the manifestations of damage is scarring which consists of fibrous tissue that physically obstructs the motion associated with breathing and reduces the area available for air and blood to mix when the red blood cells take up oxygen. Hence there is a *natural* increase in equivalent smoking history that is associated with physical age and smoking will cause a faster increase in equivalent smoking history 820 during the period between when the patient started smoking 822 and when they stopped smoking 824. Similarly, industrial exposure to chemicals such as asbestos and exposure to airborne pollution will also result in a more rapid increase in the equivalent smoking history as a patient ages than someone without such exposure. Therefore, in some examples, a smoking history parameter, such as smoking history parameter 350 in FIG. 3, may be a useful output 355 of a smoking history system 340, in isolation of any lung medical condition analysis and score and following an analysis of at least one medical image 310. In order to better account for an increased risk of lung disease from causes other than smoking, data for at-risk non-smokers can optionally be included during training. In this manner, it is envisaged that a smoking history parameter 350 may be a useful output 355 of a smoking history system 340 on its own, and will be useable by clinicians in understanding a risk of lung disease of a patient.

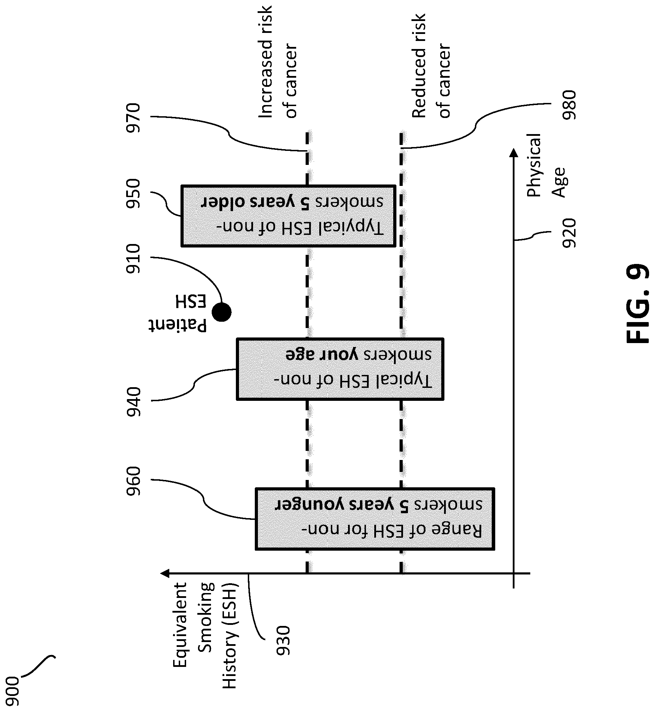

Referring now to FIG. 9, the equivalent smoking history for a patient 910 can also be a useful output of a CADx system, as the value(s) may be used to motivate them to stop smoking by for example showing their equivalent smoking history as a location 910 on a pair of axes that represent physical age 920 and equivalent smoking history 930, within the spectrum of typical equivalent smoking history of other people of the same age 940. In addition, in some examples, the spectra for other people who may be older 950 and/or younger 960 may also be determined and compared to a current patient by the CADx system, and may subsequently be presented to the patient as a comparison of how their lung appears. Finally, a region of equivalent smoking history associated with significantly increased risk of cancer 970, or reduced risk 980, may also be shown for comparison. Thereafter, in some examples, the physician may use, say, two measures of equivalent smoking history, which may be taken at separate times in order to highlight to patients when their lung age is increasing more rapidly than is normal for their physical age.

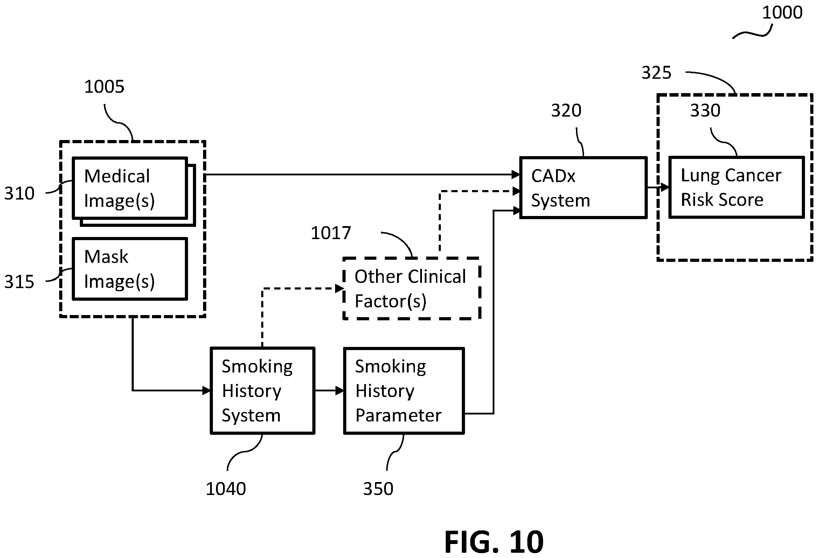

FIG. 10 illustrates a further realisation of the CADx device 1000, which uses a different approach to reduce the error in the equivalent smoking history: which is to couple the output of the smoking history system 1040 to at least one clinical factor other than smoking history 1017 in addition to smoking history 350. For example the output of a neural network (such as neural network 630 in FIG. 6) may include one scalar for the smoking history and one scalar for each of the clinical factors other than smoking history 1017. Terms for all the scalars are included in the loss function during training. Thus, the neural network may be trained to simultaneously estimate a patient's smoking history and at least one clinical factor other than smoking history 1017, which forces the neural network to include information on the clinical factor other than smoking history 1017 when estimating the risk of cancer in order to improve the accuracy of the measure. In this example, a joint loss function that penalises errors when estimating the clinical parameter may be used during training, for example (10.1):

Joint loss function:

$$J = X\left(\widehat{H}, H\right) + \sum_i (\widehat{p}_{ij} - p_{ij})^2 \qquad (10.1),$$

where the joint loss function, $J$, computes a mathematical function called the negative cross-entropy, $X$, between the actual smoking history, $\widehat{H}$, smoking history parameter, $H$, and the sum of the square differences between the estimate of the at least one additional clinical parameter other than smoking history, $\widehat{p}$. and the actual value for the at least one clinical parameter other than smoking history, $p$. In this example the training index, $i$, is unique for each example of a training image and corresponding label, within the training set, while the clinical parameter index, $j$, is used to denote one of the clinical parameters other than smoking history that is being estimated by the model.

The estimated clinical factors other than smoking history 1017 may optionally be discarded from the CADx system once training is complete.

Thus, examples of the invention provide a CADx system that can be trained, such that it optimises performance whilst taking into account the overall clinical context. Considering the example discussed, it is not necessary to correctly estimate the smoking history of all medical images to obtain clinical and economic benefit. For instance if the CADx system classifies nodules as either malignant or benign, one aim of examples of the present invention is to maximise a number of benign nodules that are correctly classified, whilst not misclassifying any malignant nodules.

Although examples of the invention have been described with reference to the CADx system being used to assist in the interpretation of chest images and lung nodules, it is envisaged that the concepts described herein may be employed beyond this area of the human body. In other examples, it is envisaged that the concepts may be applied in any medical application where it is important to consider other aspects of the clinical context, such as economic and patient preferences, where one or more medical images are being analysed.

Although examples of the invention have been described with reference to measuring lung cancer risk that estimates the smoking history of a specific patient, it is envisaged that the concepts described herein may be employed in an automated system that examines all medical images stored on, say, a hospital database, in order to identify patients with a high determined 'equivalent smoking history' who, in the absence of other information, can be assumed to be of greater risk of lung disease. Advantageously, these high 'equivalent smoking history' scans may not have been reviewed to check for lung nodules, but the patients are subsequently automatically prioritised for review in response to the automated system results.

Although examples of the invention have been described with reference to a CADx device that estimates a smoking history of a specific patient, it is envisaged that the concepts described herein may be employed by a nodule clinic (NC) manager that is reviewing a medical image for enrolment of a patient into a nodule clinic, where the patient's smoking history has never been recorded. In this context, the NC manager may use the concepts herein described to assess the patient's smoking history from the image, for use during subsequent review of the patient.

Although examples of the invention have been described with reference to a CADx device that estimates the smoking history of a specific patient, it is envisaged that the concepts described herein may be employed by, say, the device on any images that are taken of potential patients. For example, a person may undergo a chest X-Ray after a bicycle accident, and the CADx device (which may be configured to run automatically overnight) may detect the person's smoking history as being high, relative to their physical age. This information may then be sent to their GP, who may request a spirometry test. Based on the spirometry result, Chronic Obstructive Pulmonary Disease may be diagnosed and at the GP appointment it becomes clear that the patient suffers from breathlessness and frequent pneumonia. The patient may then be advised to stop smoking and given a bronchodilator to help their symptoms, as one example of a result of the CADx device that estimates the smoking history of a specific patient according to examples herein described.

Although examples of the invention have been described with reference to a CADx device, it is envisaged that the improved lung cancer risk measure may be employed by a nodule clinic (NC) manager or pulmonologist in assessing a nodule's malignancy. For example, the nodule may be of intermediate size and may appear to the NC manager to be potentially suspicious. It may also be that a lung cancer prediction (LCP) score, which was computed without smoking history, is of intermediate risk, i.e. it concurs that the nodule is not obviously benign. However, after employing the concepts herein described, the smoking history of the patient is found to be high. Inclusion of the smoking history parameter may be configured to cause the LCP score to change in order to indicate an increased risk of malignancy. As a consequence, the patient may be required to attend a follow-up check after a shorter interval, where the nodule is found to have grown. Subsequent biopsy could identify the nodule as being a progressive squamous cell carcinoma. Thanks to the shortened follow-up time, the cancer is identified early enough that a lobectomy procedure cures the patient, because further growth and secondary cancers never have a chance to occur.

Although examples of the invention have been described with reference to a CADx device, it is envisaged that a GP may use the determined smoking history parameter (or the increase in determined smoking history parameter relative to actual age) output from the CADx device to help a patient understand why they need to stop smoking in order to reduce their risk of getting lung cancer.

The present invention has been described with reference to the accompanying drawings. However, it will be appreciated that the present invention is not limited to the specific examples herein described and as illustrated in the accompanying drawings. Furthermore, because the illustrated embodiments of the present invention may for the most part, be implemented using electronic components and circuits known to those skilled in the art, details will not be explained in any greater extent than that considered necessary as illustrated above, for the understanding and appreciation of the underlying concepts of the present invention and in order not to obfuscate or distract from the teachings of the present invention.

The invention may be implemented in a computer program for running on a computer system, at least including code portions for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system. Therefore, some examples describe a non-transitory computer program product having executable program code stored therein for receiving at least one input medical image of a patient in which the patient's lungs are visible. The method further includes processing the at least one input medical image and automatically computing the patient's smoking history based on the at least one input medical image; and outputting the smoking history from the CADx device.

The computer program may be stored internally on a tangible and non-transitory computer readable storage medium or transmitted to the computer system via a computer readable transmission medium. All or some of the computer program may be provided on computer readable media permanently, removably or remotely coupled to an information processing system. The tangible and non-transitory computer readable media may include, for example and without limitation, any number of the following: magnetic storage media including disk and tape storage media; optical storage media such as compact disk media (e.g., CD-ROM, CD-R, etc.) and digital video disk storage media; non-volatile memory storage media including semiconductor-based memory units such as FLASH memory, EEPROM, EPROM, ROM; ferromagnetic digital memories; MRAM; volatile storage media including registers, buffers or caches, main memory, RAM, etc.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims and that the claims are not limited to the specific examples described above.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures can be implemented which achieve the same functionality.

Any arrangement of components to achieve the same functionality is effectively 'associated' such that the desired functionality is achieved. Hence, any two components herein combined to achieve a particular functionality can be seen as 'associated with' each other such that the desired functionality is achieved, irrespective of architectures or intermediary components. Likewise, any two components so associated can also be viewed as being 'operably connected,' or 'operably coupled,' to each other to achieve the desired functionality.

Furthermore, those skilled in the art will recognize that boundaries between the above described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms 'a' or 'an,' as used herein, are defined as one or more than one. Also, the use of introductory phrases such as 'at least one' and 'one or more' in the claims should not be construed to imply that the introduction of another claim element by the indefinite articles 'a' or 'an' limits any particular claim containing such introduced claim element to inventions containing only one such element, even when the same claim includes the introductory phrases 'one or more' or 'at least one' and indefinite articles such as 'a' or 'an.' The same holds true for the use of definite articles. Unless stated otherwise, terms such as 'first' and 'second' are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

**EP 3 878 353 B1**

**Claims**

1. A Computer Aided Diagnosis, CADx, lung disease risk measure device (300, 700, 1000) comprising:

    an input circuit (305, 610, 705, 1005) configured to receive at least one input medical image (310) of a patient in which the patient's lungs are visible;
    a smoking history system (340, 740, 1040) operably coupled to the input circuit (305, 610, 705, 1005) and configured to receive and analyse the at least one input medical image (310) of the patient and determine an equivalent smoking history of the patient based on the at least one input medical image (310) and output a smoking history parameter (350) based on the determined equivalent smoking history of the patient;
    a CADx system (320) configured to receive and analyse the smoking history parameter (350) and the at least one input medical image (310), and calculate and output a lung disease risk measure (330) based on a combination of the analysis of the at least one input medical image (310) and the smoking history parameter (350); and
    an output circuit (325) operably coupled to the smoking history system (340, 1040) and the CADx system (320) and arranged to output the lung disease risk measure (330).

2. The CAD lung disease risk measure device (300, 700, 1000) of Claim 1, wherein the smoking history system (340, 740, 1040) is configured to determine an equivalent smoking history of the patient by comparing the at least one input medical image (310) with a range of pre-stored, classified input medical images, and to determine the smoking history parameter (350) based on a quantification of cumulative damage of the patient's lungs as analysed from the at least one input medical image.

3. The CAD lung disease risk measure device (300) of Claim 1, wherein the CADx system (320) further comprises an input configured to receive at least one clinical factor (317, 1017) related to the patient and calculate and output a lung disease risk measure (330) based on a combination of the analysis of the at least one input medical image (310) and the received smoking history parameter (350) and the at least one clinical factor (380) of the patient.

4. The CADx, lung disease risk measure device (700) of Claim 1 or Claim 2, further comprising:

    a risk score adjustment system (760) coupled to an output of the CADx system (720) and an output of the smoking history system (740) and configured to adjust the received lung disease risk measure (730) based on the received smoking history parameter (350) of the patient; and
    wherein the output circuit (765) is further operably coupled to the risk score adjustment system (760) and arranged to output a modified lung disease risk measure (770).

5. The CAD lung disease risk measure device (700) of Claim 4, wherein the risk score adjustment system (760) further comprises an input configured to receive at least one clinical factor (780) related to the patient and configured to adjust the received lung disease risk measure (730) based on the received smoking history parameter (350) and the at least one clinical factor (780) of the patient.

6. The CAD lung disease risk measure device (300, 700, 1000) of Claim 3 or Claim 5, wherein the at least one clinical factor (317, 780, 1017) comprises data on at least one of: the patient's physical age, the patient's body-mass index, a diagnosed condition, a clinical measure of lung function, whether the patient is currently suffering from an infection.

7. The CAD lung disease risk measure device (300, 700) of Claim 6, wherein either the CADx system (320) or the risk score adjustment system (760) is configured to automatically compute an effective lung damage of the patient based on the at least one medical image and map the effective lung damage of the patient to a measure of a damaged lung that is equivalent to a second patient having a smoking history.

8. The CAD lung disease risk measure device (300, 700, 1000) of Claim 7, further configured to generate the measure of the damaged lung in terms of a period of time and a quantity of smoking during that time of an equivalent smoking patient.

9. The CAD lung disease risk measure device (300, 700, 1000) of any of preceding Claims 1 or 3 to 8, wherein the lung disease risk measure is a single number that provides a modified risk measure of the patient's risk of lung disease beyond a natural effect of patient aging.

10. The CAD lung disease risk measure device (300, 700, 1000) of any of preceding Claims 1 or 3 to 9, wherein at least one of: the CADx system (320) and the smoking history system (340, 740, 1040) and the risk score adjustment system (760) is implemented as a neural network (600) and they are trained jointly.

11. The CAD lung disease risk measure device (300, 700, 1000) of any of preceding Claims 1 or 3 to 10, wherein the at least one input medical image (310) comprises an associated at least one input ground-truth label (535) and wherein at least one of: the CADx system (320) and the smoking history system (340, 740, 1040) and the risk score adjustment system (760) is configured to calculate a difference between the determined equivalent smoking history of the patient (350) and a corresponding at least one input ground-truth label (535) using a loss function (560).

12. The CAD lung disease risk measure device (300, 700, 1000) of Claim 11, wherein the at least one input ground-truth label (535) comprises at least the actual smoking history of the patient and a difference between the smoking history parameter (350) and the actual smoking history is computed using a loss function during training.

**Patentansprüche**

1. Vorrichtung mit computergestützter Diagnose (Computer Aided Diagnosis - CADx; CADx-Vorrichtung) für ein Risikomaß einer Lungenerkrankung (300, 700, 1000), umfassend:

   eine Eingangsschaltung (305, 610, 705, 1005), die dazu konfiguriert ist, mindestens ein medizinisches Eingangsbild (310) eines Patienten zu empfangen, auf dem die Lungen des Patienten sichtbar sind;
   ein Raucherkarrierensystem (340, 740, 1040), das mit der Eingangsschaltung (305, 610, 705, 1005) wirkgekoppelt ist und dazu konfiguriert ist, das mindestens eine medizinische Eingangsbild (310) des Patienten zu empfangen und zu analysieren und eine äquivalente Raucherkarriere des Patienten auf Grundlage des mindestens einen medizinischen Eingangsbildes (310) zu bestimmen und einen Raucherkarrierenparameter (350) auf Grundlage der bestimmten äquivalenten Raucherkarriere des Patienten auszugeben;
   ein CADx-System (320), das dazu konfiguriert ist, den Raucherkarrierenparameter (350) und das mindestens eine medizinische Eingangsbild (310) zu empfangen und zu analysieren und auf Grundlage einer Kombination der Analyse des mindestens einen medizinischen Eingangsbilds (310) und des Raucherkarrierenparameters (350) ein Risikomaß einer Lungenerkrankung (330) zu berechnen und auszugeben; und
   eine Ausgangsschaltung (325), die mit dem Raucherkarrierensystem (340, 1040) und dem CADx-System (320) wirkgekoppelt ist und dazu angeordnet ist, das Risikomaß einer Lungenerkrankung (330) auszugeben.

2. CAD-Vorrichtung für ein Risikomaß einer Lungenerkrankung (300, 700, 1000) nach Anspruch 1, wobei das Raucherkarrierensystem (340, 740, 1040) dazu konfiguriert ist, eine äquivalente Raucherkarriere des Patienten durch Vergleichen des mindestens einen medizinischen Eingangsbilds (310) mit einem Bereich von vorab gespeicherten, klassifizierten medizinischen Eingangsbildern zu bestimmen und den Raucherkarrierenparameter (350) auf Grundlage einer Quantifizierung von kumulativer Lungenschädigung des Patienten, wie sie aus dem mindestens einen medizinischen Eingangsbild analysiert wird, zu bestimmen.

3. CAD-Vorrichtung für ein Risikomaß einer Lungenerkrankung (300) nach Anspruch 1, wobei das CADx-System (320) ferner einen Eingang umfasst, der dazu konfiguriert ist, mindestens einen klinischen Faktor (317, 1017), der mit dem Patienten in Beziehung steht, zu empfangen und ein Risikomaß einer Lungenerkrankung (330) auf Grundlage einer Kombination der Analyse des mindestens einen medizinischen Eingangsbilds (310) und des empfangenen Raucherkarrierenparameters (350) und des mindestens einen klinischen Faktors (380) des Patienten zu berechnen und auszugeben.

4. CADx-Vorrichtung für ein Risikomaß einer Lungenerkrankung (700) nach Anspruch 1 oder Anspruch 2, ferner umfassend:

   ein Risikobewertungseinstellungssystem (760), das mit einem Ausgang des CADx-Systems (720) und einem Ausgang des Raucherkarrierensystems (740) gekoppelt ist und dazu konfiguriert ist, das empfangene Risikomaß einer Lungenerkrankung (730) auf Grundlage des empfangenen Raucherkarrierenparameters (350) des Patienten einzustellen; und
   wobei die Ausgangsschaltung (765) ferner mit dem Risikobewertungseinstellungssystem (760) wirkgekoppelt und dazu angeordnet ist, ein modifiziertes Risikomaß einer Lungenerkrankung (770) auszugeben.

5. CAD-Vorrichtung für ein Risikomaß einer Lungenerkrankung (700) nach Anspruch 4, wobei das Risikobewertungseinstellungssystem (760) ferner einen Eingang umfasst, der dazu konfiguriert ist, mindestens einen klinischen Faktor (780) zu empfangen, der mit dem Patienten in Beziehung steht, und dazu konfiguriert ist, das empfangene Risikomaß einer Lungenerkrankung (730) auf Grundlage des empfangenen Raucherkarrierenparameters (350) und des mindestens einen klinischen Faktors (780) des Patienten einzustellen.

6. CAD-Vorrichtung für ein Risikomaß einer Lungenerkrankung (300, 700, 1000) nach Anspruch 3 oder Anspruch 5, wobei der mindestens eine klinische Faktor (317, 780, 1017) Daten über mindestens eines der Folgenden umfasst: das physische Alter des Patienten, den Body-Mass-Index des Patienten, einen diagnostizierten Zustand, ein klinisches Maß für die Lungenfunktion, ob der Patienten derzeit an einer Infektion leidet.

7. CAD-Vorrichtung für ein Risikomaß einer Lungenerkrankung (300, 700) nach Anspruch 6, wobei entweder das CADx-System (320) oder das Risikobewertungseinstellungssystem (760) dazu konfiguriert ist, automatisch eine effektive Lungenschädigung des Patienten auf Grundlage des mindestens einen medizinischen Bildes zu berechnen und die effektive Lungenschädigung des Patienten auf ein Maß einer beschädigten Lunge, das äquivalent zu einem zweiten Patienten mit einer Raucherkarriere ist, abzubilden.

8. CAD-Vorrichtung für ein Risikomaß einer Lungenerkrankung (300, 700, 1000) nach Anspruch 7, die ferner dazu konfiguriert ist, das Maß der beschädigten Lunge in Bezug auf einen Zeitraum und eine Rauchmenge während dieser Zeit für einen äquivalenten rauchenden Patienten zu erzeugen.

9. CAD-Vorrichtung für ein Risikomaß einer Lungenerkrankung (300, 700, 1000) nach einem der vorhergehenden Ansprüche 1 oder 3 bis 8, wobei das Risikomaß einer Lungenerkrankung eine einzelne Zahl ist, die ein modifiziertes Risikomaß für das Lungenerkrankungsrisiko des Patienten über eine natürliche Wirkung der Alterung des Patienten hinaus bereitstellt.

10. CAD-Vorrichtung für ein Risikomaß einer Lungenerkrankung (300, 700, 1000) nach einem der vorhergehenden Ansprüche 1 oder 3 bis 9, wobei mindestens eines von dem CADx-System (320) und dem Raucherkarrierensystem (340, 740, 1040) und dem Risikobewertungseinstellungssystem (760) als ein neuronales Netzwerk (600) umgesetzt ist und diese gemeinsam trainiert werden.

11. CAD-Vorrichtung für ein Risikomaß einer Lungenerkrankung (300, 700, 1000) nach einem der vorhergehenden Ansprüche 1 oder 3 bis 10, wobei das mindestens eine medizinische Eingangsbild (310) eine zugeordnete mindestens eine Ground-Truth-Eingangsmarkierung (535) umfasst und wobei mindestens eines von dem CADx-System (320) und dem Raucherkarrierensystem (340, 740, 1040) und dem Risikobewertungseinstellungssystem (760) dazu konfiguriert ist, eine Differenz zwischen der bestimmten äquivalenten Raucherkarriere des Patienten (350) und einer entsprechenden mindestens einen Ground-Truth-Eingangsmarkierung (535) unter Verwendung einer Verlustfunktion (560) zu berechnen.

12. CAD-Vorrichtung für ein Risikomaß einer Lungenerkrankung (300, 700, 1000) nach Anspruch 11, wobei die mindestens eine Ground-Truth-Eingangsmarkierung (535) mindestens die tatsächliche Raucherkarriere des Patienten umfasst und eine Differenz zwischen dem Raucherkarrierenparameter (350) und der tatsächlichen Raucherkarriere unter Verwendung einer Verlustfunktion während des Trainings berechnet wird.

**Revendications**

1. Dispositif de mesure du risque de la maladie pulmonaire de diagnostic assisté par ordinateur, CADx, (300, 700, 1000) comprenant :

un circuit d'entrée (305, 610, 705, 1005) configuré pour recevoir au moins une image médicale d'entrée (310) d'un patient dans laquelle les poumons du patient sont visibles ;
un système d'antécédents de tabagisme (340, 740, 1040) couplé de manière fonctionnelle au circuit d'entrée (305, 610, 705, 1005) et configuré pour recevoir et analyser l'au moins une image médicale d'entrée (310) du patient et déterminer des antécédents de tabagisme équivalents du patient sur la base de l'au moins une image médicale d'entrée (310) et émettre un paramètre d'antécédents de tabagisme (350) sur la base des antécédents de tabagisme équivalents déterminés du patient ;
un système CADx (320) configuré pour recevoir et analyser le paramètre d'antécédents de tabagisme (350) et l'au moins une image médicale d'entrée (310), et calculer et émettre une mesure de risque de maladie pulmonaire (330) sur la base d'une combinaison de l'analyse de l'au moins une image médicale d'entrée (310) et du paramètre d'antécédents de tabagisme (350) ; et
un circuit de sortie (325) couplé de manière opérationnelle au système d'antécédents de tabagisme (340, 1040) et au système CADx (320) et agencé pour émettre la mesure de risque de maladie pulmonaire (330).

2. Dispositif de mesure du risque de maladie pulmonaire CAD (300, 700, 1000) selon la revendication 1, dans lequel le système d'antécédents de tabagisme (340, 740, 1040) est configuré pour déterminer des antédédents de tabagisme équivalents du patient en comparant l'au moins une image médicale d'entrée (310) avec une plage d'images médicales d'entrée classifiées pré-stockées, et pour déterminer le paramètre d'antécédents de tabagisme (350) sur la base d'une quantification des dommages cumulés des poumons du patient tels qu'ils sont analysés à partir de l'au moins une image médicale d'entrée.

3. Dispositif de mesure du risque de maladie pulmonaire CAD (300) selon la revendication 1, dans lequel le système CADx (320) comprend en outre une entrée configurée pour recevoir au moins un facteur clinique (317, 1017) lié au patient et calculer et émettre une mesure de risque de maladie pulmonaire (330) sur la base d'une combinaison de l'analyse de l'au moins une image médicale d'entrée (310) et du paramètre d'antécédents de tabagisme reçu (350) et de l'au moins un facteur clinique (380) du patient.

4. CADx, dispositif de mesure du risque de maladie pulmonaire (700) selon la revendication 1 ou la revendication 2, comprenant en outre :

un système d'ajustement de score de risque (760) couplé à une sortie du système CADx (720) et à une sortie du système d'antécédents de tabagisme (740) et configuré pour ajuster la mesure de risque de maladie pulmonaire reçue (730) sur la base du paramètre d'antécédents de tabagisme reçu (350) du patient ; et
dans lequel le circuit de sortie (765) est en outre fonctionnellement couplé au système d'ajustement de score de risque (760) et agencé pour émettre une mesure de risque de maladie pulmonaire modifiée (770).

5. Dispositif de mesure du risque de maladie pulmonaire CAD (700) selon la revendication 4, dans lequel le système d'ajustement de score de risque (760) comprend en outre une entrée configurée pour recevoir au moins un facteur clinique (780) lié au patient et configurée pour ajuster la mesure de risque de maladie pulmonaire (730) reçue sur la base du paramètre d'antécédents de tabagisme reçu (350) et de l'au moins un facteur clinique (780) du patient.

6. Dispositif de mesure du risque de maladie pulmonaire CAD (300, 700, 1000) selon la revendication 3 ou la revendication 5, dans lequel l'au moins un facteur clinique (317, 780, 1017) comprend des données sur au moins l'un parmi : l'âge physique du patient, l'indice de masse corporelle du patient, une affection diagnostiquée, une mesure clinique de la fonction pulmonaire, la question de savoir si le patient souffre actuellement d'une infection.

7. Dispositif de mesure du risque de maladie pulmonaire CAD (300, 700) selon la revendication 6, dans lequel soit le système CADx (320) soit le système d'ajustement de score de risque (760) est configuré pour calculer automatiquement une lésion pulmonaire effective du patient sur la base de l'au moins une image médicale et mapper les dommages pulmonaires effectifs du patient à une mesure d'un poumon endommagé qui équivaut à un second patient ayant des antécédents de tabagisme.

8. Dispositif de mesure du risque de maladie pulmonaire CAD (300, 700, 1000) selon la revendication 7, configuré en outre pour générer la mesure du poumon endommagé en termes d'une période de temps et d'une quantité de tabagisme pendant cette période d'un patient fumeur équivalent.

9. Dispositif de mesure du risque de maladie pulmonaire CAD (300, 700, 1000) selon l'une quelconque des revendications précédentes 1 ou 3 à 8, dans lequel la mesure du risque de maladie pulmonaire est un nombre unique qui fournit une mesure de risque modifiée du risque de maladie pulmonaire du patient au-delà d'un effet naturel du vieillissement du patient.

10. Dispositif de mesure du risque de maladie pulmonaire CAD (300, 700, 1000) selon l'une quelconque des revendications précédentes 1 ou 3 à 9, dans lequel au moins l'un parmi : le système CADx (320) et le système d'antécédents de tabagisme (340, 740, 1040) et le système d'ajustement de score de risque (760) est mis en œuvre sous la forme d'un réseau neuronal (600) et ils sont entraînés conjointement.

11. Dispositif de mesure du risque de maladie pulmonaire CAD (300, 700, 1000) selon l'une quelconque des revendications précédentes 1 ou 3 à 10, dans lequel l'au moins une image médicale d'entrée (310) comprend au moins une étiquette de vérité de base d'entrée associée (535) et dans lequel au moins l'un parmi : le système CADx (320) et le système d'antécédents de tabagisme (340, 740, 1040) et le système d'ajustement de score de risque (760) est configuré pour calculer une différence entre les antécédents de tabagisme équivalents déterminés du patient (350) et au moins une étiquette de vérité de base d'entrée correspondante (535) utilisant une fonction de perte (560) .

12. Dispositif de mesure du risque de maladie pulmonaire CAD (300, 700, 1000) selon la revendication 11, dans lequel l'au moins une étiquette de vérité de base d'entrée (535) comprend au moins les antécédents de tabagisme réels du patient et une différence entre le paramètre d'antécédents de tabagisme(350) et les antécédents de tabagisme réels est calculée à l'aide d'une fonction de perte pendant l'entraînement.

**FIG. 1**

**FIG. 2**

**FIG. 3**

EP 3 878 353 B1

FIG. 4

**FIG. 5**

**FIG. 6**

Node mapping function:
$$d = A(w_{ad} \times a + w_{bd} \times b + w_{cd} \times c)$$

**FIG. 7**

EP 3 878 353 B1

**FIG. 8**

EP 3 878 353 B1

**FIG. 9**

**FIG. 10**

EP 3 878 353 B1